# EUROPEAN PATENT APPLICATION

(11) **EP 1 646 131 A1**
(43) Date of publication of application: **12.04.2006**
(21) Application number: 04733434.7
(22) Date of filing: 17.05.2004
(51) Int. Cl.: H02K 23/54

(54) **DC MOTOR**

(30) Priority: 19.05.2003 JP 2003140329
(71) Applicant: Kabushiki-Kaisha Asaba, Tokyo 130-0013 (JP)
(72) Inventor: ASABA, Keisuke, c/o Kabushiki Kaisha Asaba, Tokyo 1300013 (JP); SEKINE, Takehiro, c/o Kabushiki Kaisha Asaba, Tokyo 1300013 (JP)
(74) Representative: Curtis, Philip Anthony
(86) International application number: PCT/JP2004/006630
(87) International publication number: WO 2004/102774

(57) **Abstract**

ADC motor with an armature coil for generating rotational torque capable of improving motor efficiency and generated torque while achieving size and thickness reductions, comprising magnets 23A to 23D and armature coils 31A to 31C, 32A to 32C, wherein, for example, a magnet portion is used as a rotor 12. A stator 13 at an armature coil portion comprises an inner coil group formed by arranging, parallel with each other, hollow inner coil bodies 31A to 31C on peripheral side surfaces of a magnet yoke 22 and the magnets 23A to 23D as a virtual disc by a prescribed quantity and an outer coil group formed by arranging, parallel with each other, a prescribed number of hollow outer coil bodies 32A to 32C while covering the inner coil group. The peripheral side surface of the inner coil group is made externally flush with the peripheral side surface of the outer coil group.

## Description

### Technical Field

The present invention relates to a DC motor having armature coils producing rotational torques.

### Background Art

In these years, various types of DC motors depending upon applications have been known which ranges from very small-sized types used in a clock or the like to medium-sized and large-sized types used in a power system for an electric bicycle, an electric vehicle or the like. Such a DC motor has a fundamental issue of efficiency improvement of rotation and torque generation in any application. Furthermore, in using a DC motor, there is a request for higher response speed.

DC motors can be classified into brush motors, brushless motors, cored motors, coreless motors and so on. The cored motors can be classified into slotted motors and slotless motors. Each of such DC motors has a fundamental configuration consisting of magnets constituting a magnetic field structure and armature coils constituting an armature structure, and is driven with one of them used as a rotor and the other used as a stator depending upon usage of them. In this case, the following patent documents are known as a structure of the armature coil.

Patent Document 1: Domestic Re-publication of PCT International Application No. 00-062401
Patent Document 2: Japanese Patent Laid-Open No. 60-241761

The above-mentioned Patent Document 1 disclosures an inner rotor type brushless motor disposed with three hollow armature coils, outer peripheries of which are fitted to each other and toruses are wound in line parallel to the diametrical directions of ring-shaped magnets so as to fit the outer periphery of the armature. This brushless motor can achieve size and thickness reductions, high torque generation efficiency and energy saving.

The above-mentioned Patent Document 2 discloses a slotless motor, of which torus is wound with an armature coil into a dogleg shape of 120 degrees so as to fit an outer periphery of the armature coil to an outer periphery of a ring-shaped layered yoke. Such a slotless motor is shown as the one that makes almost all portions of a coil winding contribute to torque generation.

Furthermore, an inner rotor type brushless motor is also known which uses a hollow coil concentrically winding a torus so as to fit an outer periphery of the armature coil to an outer periphery of a ring-shaped magnet.

### Disclosure of the Invention

### Problems to be Solved by the Invention

In a case where an armature coil rotates with the armature coil serving as a hollow coil (coreless motor), each other's adhesive portion of the armature coils is restricted, therefore dropping-off may occur due to centrifugal force, impact or the like. Where a magnet (rotor) rotates, bearings for rotating a shaft are required to be mounted in the center thereof, thus causing such problems as the number of windings or the size of the armature coil has a limit thereof and a response speed during rotation cannot be improved.

A coreless motor generally uses a magnet with two poles. In a magnetic circuit of a housing, magnetism is apt to be saturated because a path in which the magnetism returns is long. This requires an increase in the thickness of a magnetic material, thus causing a problem of miniaturization being inhibited. In most cases, a magnet with two poles and two brushes 180 degrees spaced from each other are used and only coil control for each 180-degree is performed, thus causing such a problem that efficiency improvement is difficult.

On the other hand, a generated torque can be improved by the thickness or the number of turns of an armature coil, however, if the thickness of the armature is set to be twice, for example, a gap between a magnet and a yoke becomes twice, thus degrading torque generation efficiency. Accordingly, there exists a proposition that torque generation efficiency by the armature coil should be improved while the gap between the magnet and the yoke is being kept small.

In view of the aforementioned problems, it is an object of the present invention to provide a DC motor capable of size and thickness reductions and improvements in motor efficiency, generated torque and response.

### Means for Solving the Problem

To solve the above-mentioned problems, a DC motor according to Claim 1 includes magnets as a main source for generating a magnetic flux and armature coils as a main source for generating a torque and using either thereof as a rotor, wherein the armature coil comprises an inner coil group formed by arranging, parallel with each other, a prescribed number of hollow inner coil bodies of a prescribed shape wound with a conductor of a prescribed number of turns on peripheral side surfaces of a virtual disc or a disc-shaped core; and an outer coil group formed by arranging, parallel with each other, a prescribed number of hollow outer coil bodies of a prescribed shape wound with a conductor of a prescribed number of turns on peripheral side surfaces with the inner coil group taken as a virtual disc.

The DC motor according to Claims 2 to 6 is structured as follows:
"The peripheral side surface of the inner coil group is made externally flush with the peripheral side surface of the outer coil group";
"The respective inner coil bodies and the respective coil bodies are formed into a hollow and roughly trapezoidal or a hollow and arrowed shape, each of the corresponding inner coil bodies is arranged at intervals of 120 degrees, and wherein each of the corresponding outer coil bodies is shifted from each of the corresponding inner coil bodies by 60 degrees and arranged at intervals of 120 degrees.";
"Phases facing between the inner coil bodies and the outer coil bodies are connected in series or in parallel to each other, each of which is star-connected."
"In a case where the armature coil portion is made to serve as a rotor, the DC motor includes commutators adaptable to the respective inner coil bodies and the respective outer coil bodies and four brushes arranged at intervals of 90 degrees for the respective commutators."; and
"In a case where the armature coil portion is made to serve as a rotor, the DC motor includes commutators adaptable to respective coil bodies formed by star-connecting the respective inner coil bodies and the respective outer coil bodies and two brushes arranged at intervals of 90 degrees for the respective commutators."

The DC motor has magnets and armature coils, either of which is used as a rotor.
The armature coil comprises an inner coil group formed by arranging, parallel with each other, a prescribed number of hollow inner coil bodies of a prescribed shape on peripheral side surfaces of a virtual disc or a disc-shaped core; and an outer coil group formed by arranging, parallel with each other, a prescribed number of hollow outer coil bodies while covering the inner coil group. Accordingly, a minimum space can increase the number of turns of the armature coil, thus high motor efficiency and generated torque improvement can be achieved in addition to size and thickness reductions.

In a case where a magnet is disposed at hollow portions of the inner coil group and the outer coil group as a rotor, the inner coil group and the outer coil group forms such a shape as to envelope the magnet to a maximum extent, thus the rotor can be structured so as to have small inertia for high response speed.

### Advantages of the Invention

A DC motor of the present invention uses either of magnets or armature coils provided as a rotor. The armature coil comprises an inner coil group formed by arranging, parallel with each other, a prescribed number of hollow inner coil bodies of a prescribed shape on peripheral side surfaces of a virtual disc or a disc-shaped core; and an outer coil group formed by arranging, parallel with each other, a prescribed number of hollow outer coil bodies while covering the inner coil group. As a result, size and thickness reductions and high motor efficiency can be achieved and generated torque can be improved for high response speed.

By connecting, in parallel or in series, the respective facing coil bodies of the respective inner coil bodies and the respective outer coil bodies, total armature coil resistance can be selected, so that variance in applied voltage can be accommodated and a range for selecting the thickness of a wire which facilitates formation of a coil body suited to applied voltage becomes wider, thus improving the flexibility of coil body formation.

### Brief Description of the Drawings

Figure 1A is a structural view (1) of a DC motor according to a first embodiment of the present invention;
Figure 1B is a structural view (2) of a DC motor according to a first embodiment of the present invention;
Figure 1C is a structural view (3) of a DC motor according to a first embodiment of the present invention;
Figure 2 is a partial cross-section view of the DC motor illustrated in Figures 1A to 1C;
Figure 3A is a descriptive view (1) of an armature coil of the DC motor illustrated in Figures 1A to 1C;
Figure 3B is a descriptive view (2) of an armature coil of the DC motor illustrated in Figures 1A to 1C;
Figure 3C is a descriptive view (3) of an armature coil of the DC motor illustrated in Figures 1A to 1C;
Figure 4A is a descriptive view (1) of driving timings of the D C motor illustrated in Figures 1A to 1C;
Figure 4B is a descriptive view (2) of driving timings of the DC motor illustrated in Figures 1A to 1C;
Figure 4C is a descriptive view (3) of driving timings of the DC motor illustrated in Figures 1A to 1;
Figure 5A is a structural view (1) of a DC motor according to a second embodiment of the present invention;
Figure 5B is a structural view (2) of a DC motor according to a second embodiment of the present invention;
Figure 5C is a structural view (3) of a DC motor according to a second embodiment of the present invention;
Figure 6A is a structural view (1) of a DC motor according to a third embodiment of the present invention;
Figure 6B is a structural view (2) of a DC motor according to a third embodiment of the present invention;
Figure 6C is a structural view (3) of a DC motor according to a third embodiment of the present invention;
Figure 7A is a descriptive view (1) of driving timings of the DC motor illustrated in Figures 6A to 6C;
Figure 7B is a descriptive view (2) of driving timings of the DC motor illustrated in Figures 1A to 1C;
Figure 8A is a structural view (1) of a DC motor according to a fourth embodiment of the present invention;
Figure 8B is a structural view (2) of a DC motor according to a fourth embodiment of the present invention;
Figure 8C is a structural view (3) of a DC motor according to a fourth embodiment of the present invention;
Figure 9A is a descriptive view (1) of driving timings of the DC motor illustrated in Figures 8A to 8C; and
Figure 9B is a descriptive view (2) of driving timings of the DC motor illustrated in Figures 8A to 8C.

### Description of Symbols

- 11, 41, 71, 101: DC motor
- 12: Rotor
- 13: Stator
- 16: Coil yoke
- 22: Magnet yoke
- 23A to 23D: Magnet
- 31A to 31: Inner coil body
- 32A to 32C: Outer coil body

### Best Mode for Carrying Out the Invention

The following description will explain the present invention with reference to the drawings illustrating some embodiments thereof. A DC motor of the present invention is applicable to any of bush type motors, brushless motors, cored motors, coreless motors and the like. This embodiment describes the DC motor, using inner rotor type and outer rotor type brushless motors and a brushtype coreless motor as examples. The DC motor according to the present invention includes, besides the above mentioned motors, rotary actuators and the like which the same principles.

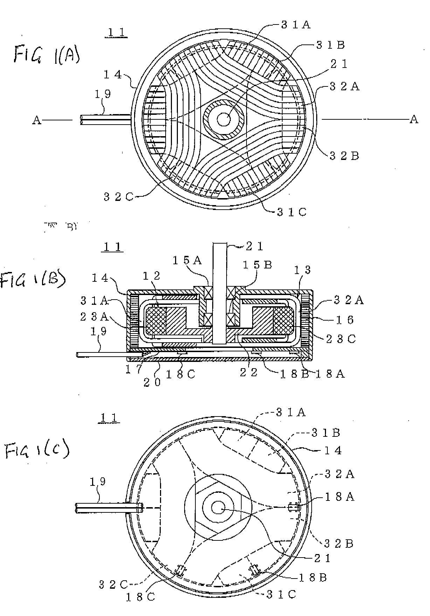

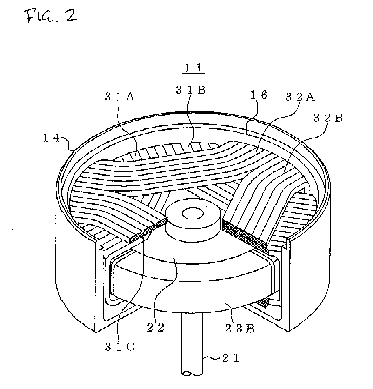

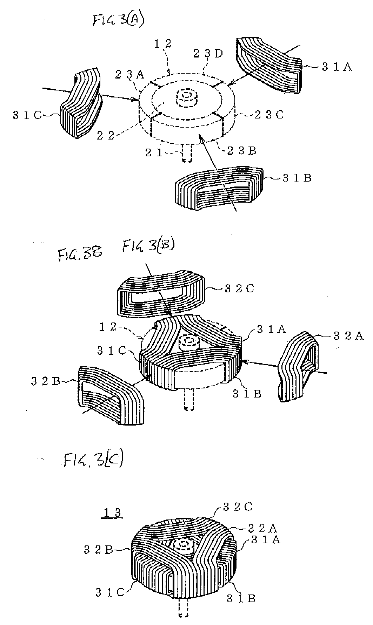

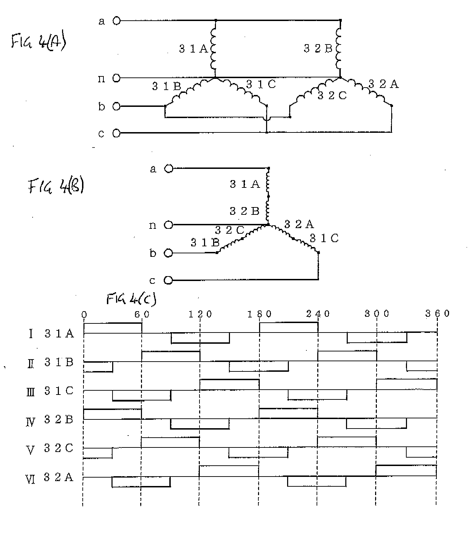

Figures 1A to 1C illustrate a structural view of a DC motor according to a first embodiment of the present invention respectively, and Figure 2 illustrates a partial perspective sectional view of the DC motor illustrated in Figures 1A to 1C. FIGS 1A to 1C and Figure 2 illustrates an inner rotor type brushless motor. Figure 1A is a structural view from the top of an inner housing, Figure 1B is a cross-sectional view of an inner housing in Figure 1A through A-A and Figure 1C is a structural view from the bottom without a cover section. As illustrated in FIGS 1A to 1C and Figure 2, a DC motor 11 is disposed with a stator 13 so as to include a rotor 12 and is stored in a housing 14. The housing 14 is of a cylindrical shape, of which one side is open and the central position of the other is formed with a cylindrical journal section.

The rotor 12, of which a shaft 21 is firmly fixed on an arm shaped magnet yoke 22, contactless secured with, for example, four (4 pole) magnets 23A to 23D for magnetic flux generation on an outer periphery of the magnet yoke 22. The shat 21 is rotatbly installed on the journal portion of the housing 14 by bearings (e.g. ball bearings) 15A, 15B. As the magnets 23A to 23D, anisotropic magnets such as Nd-iron magnets or samarium-cobalt magnets are used, where N-pole and S-pole are alternately arranged to be polarized into four poles.

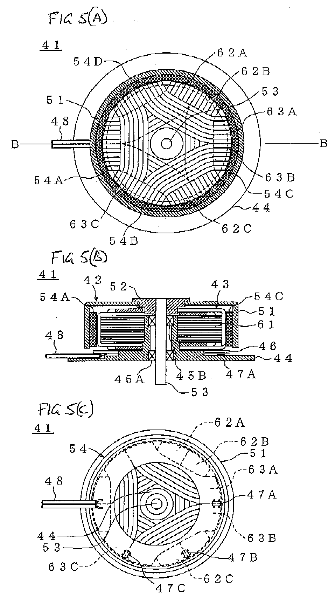

The stator 13 is formed out of a cage-typed armature coil for torque generation and consists of an inner coil group in which hollow inner coil bodies 31A to 31C of roughly trapezoidal shape (bowed shape allowable) formed by winding a conductor coil by a prescribed number of turns are arranged in parallel on a peripheral surface of the rotor 12 serving as a virtual disc; and an outer coil group in which hollow outer coil bodies 32A to 32C of roughly trapezoidal shape (bowed shape allowable) formed by winding a conductor coil by a prescribed number of turns covers the inner coil group and are arranged in parallel on a peripheral surface with the inner coil group serving as a virtual disc. (The details will be described in Figure 3.)

The stator 13 is disposed at a micro clearance (or abuts) to a coil yoke (laminated yoke) mounted on a side inner wall of the housing 14 and a printed circuit board 17 is provided under the stator 13 (on the opening side of the housing 14). The printed circuit board 17 is installed with at least hall elements 18A to 18C at specified positions (e.g. intervals of 60 degrees) and, lead wires 19 are extended to the outside of the housing 14 by a corresponding quantity. In other words, energization control is performed for the inner coil group and the outer coil group in response to detection of the hall elements 18A to 18C by an external control circuit. A roughly closed state is formed by attaching a cover 20 onto an open portion of the housing 14. On the printed circuit board 17, the control circuit may be installed together with the hall elements 18A to 18C.

Figures 3A to 3C illustrate descriptive views of armature coils of the DC motor illustrated in Figures 1A to 1C. As illustrated in Figure 3A, each of inner coil bodies 31A to 31C has a multi-layer structure formed out of normal winding wound in a round trip and formed into a hollow and roughly trapezoidal shape (bowed shape allowable). By forming the roughly trapezoidal or bowed shape, layout positions of the journal portion of the housing 14 and a commutator (brush) described later can be ensured. For outer coil bodies 32A to 32C, the shape thereof is in the same manner as the inner coil bodies, except in sizes different from each other.

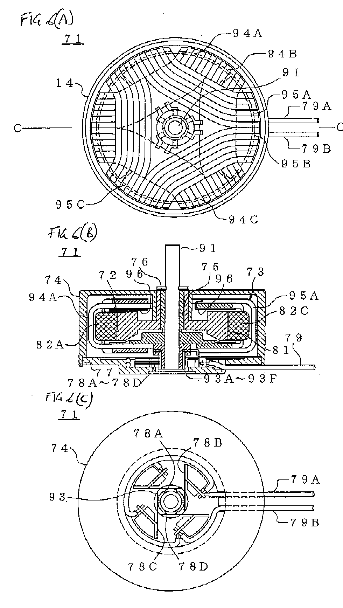

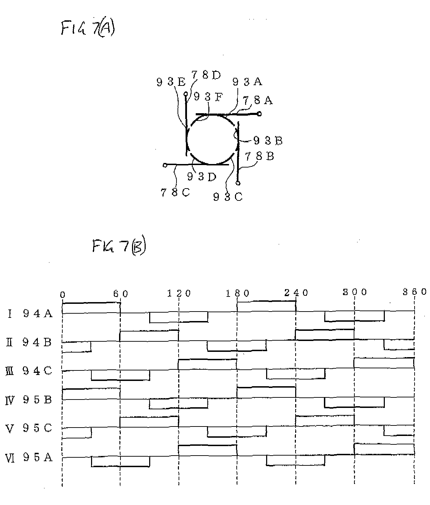

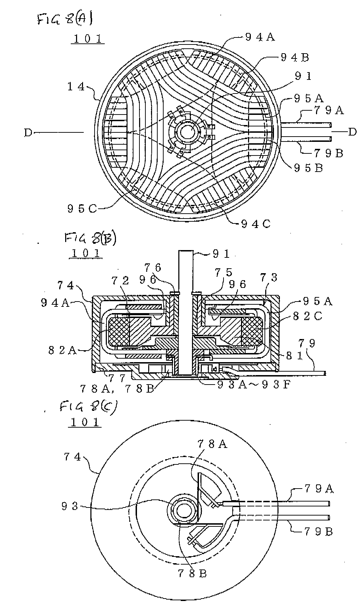

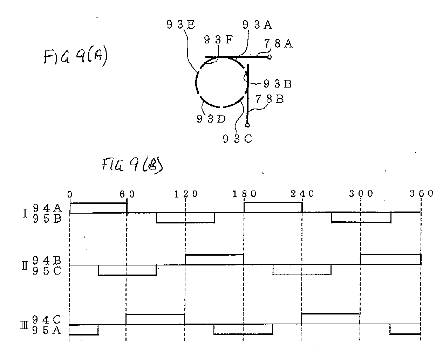

As illustrated in Figure 3A, each of the three inner coil bodies 31A to 31C is arranged in parallel at intervals of 120 degrees on a peripheral side surface of the rotor 12, taking the rotor 12 as a virtual disc, so as to form an inner coil group as illustrated in Figure 3B Each of the outer coil bodies 32A to 32C is arranged in parallel at intervals of 120 degrees, being shifted by 60 degrees from the inner coil bodies 31A to 31C, on a peripheral side surface with the inner coil group as a virtual disc, so as to form an outer coil group as illustrated in Figure 3C. In this case, the peripheral side surface of the inner coil group is made externally flush with the peripheral side surface of the outer coil group. Connections of the inner coil bodies 31A to 31C and the outer coil bodies 32A to 32C are illustrated in Figure 4.

Assembly of the DC motor 11 will be briefly described below. A shaft 21 is, first, pressed into a magnet yoke 22 and magnets 23A to 23D polarized in the above way are bonded onto the outer periphery of the magnet yoke 22. Furthermore, a coil yoke 16 is inserted into a side inner wall of the housing 14 to be bonded and bearings 15A, 15B are mounted onto a journal portion.

Then, the rotor 12 constituted of the magnets 23A to 23D (magnet yoke 22) is included in a micro gap, each of the inner coil bodies 31A to 31C is arranged and bonded at an abutting portion therebetween. Each of the outer coil bodies 32A to 32C is arranged so as to cover the inner coil bodies 31A to 31C and bonded at an abutting portion therebetween and an abutting portion against each of the inner coil bodies 31A to 31C. This forms a coil assembly of the stator 13 including the rotor 12. In assembling the inner coil bodies 31A to 31C and the outer coil bodies 32A to 32C, an appropriate fixture is used.

The coil assembly is inserted into the housing 14 (the shaft 21 is pressed into the bearings 15A, 15B of the journal portion of the housing 14) and an inner wall of a top surface (on the opposite side to an opening) of the housing 14 and upper portions of the outer coil bodies 32A to 32C are bonded to each other. The printed circuit board 17 installed with at least the hall elements 18A to 18C are attached by soldering or the like and the cover section 20 is attached onto the opening of the housing 14 by caulking or the like.

Figures 4A to 4C illusrate a descriptive view of driving timings of the DC motor illustrated in Figures 1A to 1C respectively. Figures 4A and 4B illustrate connections of the inner coil bodies 31A to 31C and the outer coil bodies 32A to 32C respectively. Figure 4C illustrates a timing chart of driving signals to the respective coil bodies.

As illustrated in Figure 4A, each of the respective inner coil bodies 31A to 31C of the inner coil group is star-connected and each of the respective outer coil bodies 32A to 32C of the outer coil group is also star-connected. The inner coil group and the outer coil group are connected in parallel so that phases may face each other (a-phase - c-phase and a neutral point n). That is, the inner coil body 31A and the outer coil body 32B (a-phase), the inner coil body 31B and the outer coil body 32C (b-phase), the inner coil body 31C and the outer coil body 32A (c-phase) and the two neutral points n are connected with each other respectively.

Figure 4B illustrates that the inner coil body 31A and the outer coil body 32B (a-phase), the inner coil body 31B and the outer coil body 32C (b-phase) and the inner coil body 31C and the outer coil body 32A (c-phase) are connected in series to each other respectively, which are star-connected (neutral point n).

By connecting, in parallel or in series, the respective facing coil bodies of the respective inner coil bodies 31A to 31C and the respective outer coil bodies 32A to 32C, total armature coil resistance can be selected, so that variance in applied voltage can be accommodated and a range for selecting the thickness of a wire which facilitates formation of a coil body suited to applied voltage becomes wider, thus improving the flexibility of coil body formation.

In the DC motor 11, the hall elements 18A to 18C are arranged at intervals of 60 degrees, and the polarities of magnetic fluxes from the rotating 4-pole magnets 23A to 23D change with rotation at every 90 degrees, so that the polarities which the hall elements 18A to 18C detect change each time the magnets 23A to 23D rotate by 30 degrees- Thus, energization control is performed at the a-phase to the c-phase in response to detection of the hall elements.

That is, as illustrated in Figure 4C, based on detection of the hall elements 18A to 18C at every 30 degrees in response to rotation of the magnets 23A to 23D, the inner coil body 31A and the outer coil body 32B (I, IV), and the inner coil 31B and the outer coil body 32C (II, V) are driven, for example, by supplying driving electric power to a-phase to b-phase. By supplying driving electric power to the a-phase to c-phase, the inner coil body 31A and the outer coil body 32B (I, IV), and the inner coil 31C and the outer coil body 32A (III, VI) are driven. By supplying driving electric power to b-phase to c-phase, the inner coil body 31B and the outer coil body 32C (II, V), and the inner coil 31C and the outer coil body 32A (III, VI) are driven.

By forming the armature coil out of the inner coil bodies 31A to 31C and the outer coil bodies 32A to 32C in the above way, a minimum space can increase magnetic fluxes generated by increasing the number of windings of the armature coil, thus providing size and thickness reductions. Especially, by taking peripheral side surfaces of the inner coil group and the outer coil group as the identical outer periphery even if the armature coil has a double structure, a gap between the yoke and the magnet can be made the same as a single structure using only a conventional inner coil group, for example, thus improving motor efficiency and generated torque. Moreover, the magnets 23A to 23D as a rotor at hollow portions of the inner coil bodies 31A to 31C and the outer coil bodies 32A to 32C has a such a shape as to be enveloped with the inner coil bodies 31A to 31C and the outer coil bodies 32A to 32C and become a structure with a low inertia as a rotor, thus improving a response speed.

Figures 5A to 5C illustrate a structural view of a DC motor according to a second embodiment of the present invention. Figures 5A to 5C illustratge an outer rotor type brashless motor. Figure 5A illustrates a structural view of a motor inside from the top, Figure 5B illustrates a cross-sectional view through B·B line and Figure 5C illustrates a structural view from the bottom with a base removed. In Figures 5A to 5C, the DC motor 41 is disposed such that the rotor 42 includes the stator 43 and attached onto the base 44 through the bearings 45A, 45B. The base 44 is formed with a cylindrical journal portion at a disc-shaped central portion, and attached with the bearings 45A, 45B on an inner wall of the journal portion.

The rotor 42 is formed with a boss 52 at a central portion of an inverted arm shaped magnet yoke 51, and the boss 52 is fixed with the shaft 53. A side inner wall of the magnet yoke 51 is fied with 4-pole magnets 54A to 54D as a main source for producing magnetic fluxes, similar to the above. The magnets 54A to 54D are similar to the magnets 23A to 23D illustrated in Figures 1A to 1C.

The stator 43 is formed with a disc-shaped laminated core 61 on an outer periphery of the journal portion of the base 44 and, on a peripheral side surface of the laminated core 61, the inner coil group formed out of the hollow inner coil bodies 62A to 62C and the outer coil group formed out of the hollow outer coil bodies 63A to 63C as illustrated in Figure 3A to 3C are mounted so as to cover the inner coil group.

In other words, as illustrated in Figures 3A to 3C, each of the three inner coil bodies 62A to 62C is arranged in parallel at intervals of 120 degrees on a peripheral side surface of the laminated core 61 to form an inner coil group. On a peripheral side surface with the inner coil group as a virtual disc, each of the three outer coil bodies 63A to 63C is shifted by 60 degrees from the inner coil bodies 31A to 31C as to cover the inner coil group and arranged in parallel at intervals of 120 degrees to form an inner coil group, and the peripheral side surface of the inner coil group is made externally flush with the peripheral side surface of the outer coil group. The inner coil bodies 62A to 62C and the outer coil bodies 63A to 63C are star-connected as illustrated in Figure 4A or Figure 4B so that phases facing each other are connected with each other.

The printed circuit board 46 is provided above the base 44 and under the stator 43. The printed circuit board 46 is installed with the hall elements 47A to 47C, for example, at intervals of 60 degrees and a corresponding number of lead wires 48 are extended to the outside.

Assembly of the DC motor 41 will be briefly described below. The boss 52 is fixed on the magnet yoke 51 by caluking or the like and the shaft 53 is pressed into the boss 52. The magnets 54A to 54D polarized in the above way is arranged with no contact of each other on a side inner wall of the magnet yoke 51 for bonding. Each of the inner coil bodies 62A to 62C is arranged on the laminated yoke 61 and bonded at abutting portions thereof and an abutting portion against the laminated yoke 61.

Each of the outer coil bodies 63A to 63C is arranged so as to cover the inner coil bodies 62A to 62C and bonded at abutting portions thereof and abutting portions with each of the inner coil bodies 31A to 31C. This forms a coil assembly of the statolr 43 enveloping the laminated yoke 61.

Next, the printed circuit board 44 installed with at least the hall elements 47A to 47C and connected with a corresponding number of lead wires 48 is soldered to the coil assembly by tapping or the like for positive attachment. The bearings 45A, 45B are attached onto the base 44 and the coil assembly is bonded to an outer periphery of the journal portion of the base 44. Then, the shaft 53 of the rotor 42 is pressed into the bearings 45A, 45B.

Driving the rotation of the DC motor 41 is the same as for the above-mentioned DC motor 11. That is, as described above, the armature coil is formed out of the inner coil bodies 62A to 62C and the outer coil bodies 63A to 63C, so that a minimum space increases the number of windings of the armature coil and increases the number of generated magnetic fluxes. Thus, besides size and thickness reductions, high motor efficiency and response speed and improvement in generated torque can be attained.

Figures 6A to 6C illustrate a structural view of a DC motor according to a third embodiment. Figures 6A to 6C illustrate an outer rotor type coreless motor with brush respectively, and Figure 6A is a structural view of the coreless motor with a brush base removed from the bottom, Figure 6B is a cross-sectional view of Figure 6B through C-C line and Figure 6C is a structural view of the brush base. In FIGS 6A to 6C, the DC motor 71 is disposed with a rotor 73 so as to include a stator 72 and accommodated in a housing 74. The housing 74 is cylindrical, of which one side is open and of which a central portion of the other is formed with a cylindrical journal portion.

In the stator 72, a disc-shaped magnet yoke 81 is fixedly installed on an inner wall of the journal portionand, for example, four magnets 82A to 82D as a main source for producing magnetic fluxes are fixedly installed on an outer periphery of the magnet yoke 81. The magnets 82A to 82D are as described above.

In the rotor 73, a shaft 91 is fixedly installed, passing through the center of a disc-shaped hub 92 and, for example, six commutators 93A to 93F (6 segments) are fixedly installed on the hub 92 on one side of the shaft 91. With the stator 72 and the hub 92 taken as an vertual disc, an inner coil group formed out of hollow inner coil bodies 94A to 94C and an outer coil group formed out of hollow outer coil bodies 95A to 95C are provided, covering the inner coil group as illustrated in Figures 3A to 3C.

That is, as illustrated in Figures 3A to 3C, each of the three inner coil bodies 94A to 94C is arranged in parallel on a peripheral side surface with the stator 72 and the hub 92 taken as a virtual disc to form an inner coil group. Each of the three outer coil bodies 95A to 95C is shifted from the inner coil bodies 94A to 94C by 60 degrees so as to cover the inner coil group and arranged in parallel at intervals of 120 degrees to form an outer coil group. The peripheral side surface of the inner coil group is made externally flush with the peripheral side surface of the outer coil group. The inner coil bodies 94A to 94C and the outer coil bodies 95A to 95C are star-connected as illustrated in Figure 4A or Figure 4B, of phases facing each other are connected.

The inner coil group and the outer coil group are reinforcingly fixed by a prescrined number of reinforcing rings 96. The reinforcing rings 96 are provided positively to be left during rotation of the armature coil. The inner coil bodies 94A to 94C and the outer coil bodies 95A to 95C are originally bonded by adhesives or the like, which are installed more fixedly than bonding between the coil bodies disclosed in Patent Document 1, therefore prevention of the coil bodies from being left due to rotation is reinforced.

A shaft 91 is rotatbly installed on an inner wall of the journal portion of the housing 74 by bearings (sleeve bearings, ball bearings or the like may be used) 75 and is provided with an E-ring 76 at a portion extending from the housing 74 of the shaft 91.

Moreover, a brush bases 77 serving as a cover section of the housing 74 is provided on the opening side of the housing 74, and four brushes 78A to 78D are arranged at corresponding brush fixing portions at intervals of 90 degrees and fixed so as not to widen by attributting against the commutators 93A to 93F. Both of the bushes 78B, 78C are connected with lead wires 79A in a connected state and both of the brushes 78D, 78A are connected with lead wires 79B in a connected state. The brush base 77 is firmly fitted into an opening portion of the housing 74 so as to roughly seal the housing 74.

Assembly of the DC motor 71 will be briefly described below. The bearing 75 are pressed into (or bonded to) the magnet yoke 81, and the magnets 82A to 82D polarized as desribed above are contactless-arranged and bonded on an outer periphery of the magnet yoke 81. Moreover, the hub 77 is pressed into the shaft 91, the magnets 82A to 82D are enveloped in a micro gap, and the inner coil bodies 91A to 94C are bonded to the hub 77 (a dedicated fixture is used). Furthermore, each of the outer coil bodies 95A to 95C is arranged so as to cover the inner coil bodies 94A to 94C as described above, bonded at abutting portions therebetween and at portions abutting against each of the inner coil bodies 94A to 94C, and the inner coil group and the outer coil group are bonded to each other by the reinforcing ring 96.

Furthermore, the commutators 93A to 93F are installed on the shaft 91, a tap on one side of the corresponding inner coil bodies 94A to 94C and the outer coil bodies 95A to 95C is jointed to risers of the commutators 93A to 93F by soldering or the like, and a tap on the other is firmly fixed to the reinforcing ring 96 by soldering or the like. Moreover, a magnet yoke 81 is inserted into a journal portion inner wall of the housing 74 for bonding.

Moreover, the brushes 78A to 78D are mounted on the brush base 77 and corresponding ones are connected to each other. The corresponding lead wires 79A, 79B are connected by soldering or the like and the brush base 77 is mounted on the housing 74 while the brushes 78A to 78D are being widened. The lead wires 79A, 79B are connected to a power supply to feed electric current and, while the brush base 77 is being rotated, they are mounted on the housing 74 according to an appropriate timing position by caulking, adhesives or the like.

Figures 7A and 7B illustrate a descritive view of driving timings of the DC motor illustrated in Figures 6A to 6C. Figure 7A is a descriptive view illusrating commutators and brushes and Figure 7B illustrates a timing chart of driving signals to respective coil bodies.

As illusrated in Figure 7A, the respective inner coil bodies 94A to 94C and the respective outer coil bodies 95A to 95C are star-connected as illusrated in Figures 4A or 4B and respective phases theof are connected to each of the commutators 93A to 93F of six segments. The brushes 78A to 78D arranged at intervals of 90 degrees are made to abut against the commutators 93A to 93F respectively.

For the DC motor 71, as illustrated in Figure 7B, for example, positive and negative voltages are alternately applied to the brushes 78A, 78C and the brushes 78B, 78D, so that driving power is alternately supplied to between a-phase and b-pahse (I, II, IV, V) of the commutators 93A, 93D (inner coil body 94A, outer coil 95B) and the commutators 93B, 93E (inner coil body 94B, outer coil 95C), between a-phase and c-pahse (I, III, IV, VI) of the commutators 93A, 93D (inner coil body 94A, outer coil 95B) and the commutators 93C, 93F (inner coil body 94C, outer coil 95A) and between b-phase and c-pahse (II, III, V, VI) of the commutators 93B, 93E (inner coil body 94B, outer coil 95C) and the commutators 93C, 93F (inner coil body 94C, outer coil 95A), respectively, thus rotating the rotor 73.

That is, the magnets 82A to 82D are polarized into 4 poles. The brushes 78A to 78D are arranged on the commutators 93A to 93Fof six segments at intervals of 90 degrees and, by alternately applying positive and negative voltages to the brushes 78A, 78C and the brushes 78B, 78D as described above, the respective inner coil bodies 94A to 94C and the respective outer coil bodies 95A to 95C switch electric currents four times each time the rotor 73 makes one turn. Accordingly, the two brushes at an interval of 180 degrees are used for the conventional two-split magnet polarized into two poles, and a magnetic circuit can be shortened more than in the case of a coreless motor constituted of one coil group (the present invention has a double structure of an inner coil group), thus providing weight reduction by decreasing the thickness of a magnetic material and energy saving by high coil control efficiency.

Even in the case of an outer rotor type coreless motor with brush equipped with commutators of six segments, the armature coil is constituted of the inner coil bodies 94A to 94C and the outer coil bodies 95A to 95C, by which a minimum space can increase the number of windings of the armature coil in the same way to increase the number of generated magnetic fluxes, thus providing size and thickness reductions. Especially, even if an armature coil used has a double structure, by setting peripheral side surfaces of the inner coil group and the outer coil group at the identical outer periphery as described above, a gap between the housing and the magnet can be made the same as a conventional single structure using only an inner coil group, thus improving motor efficiency and generated troque.

There is provided such a shape as to envelope the magnets 82A to 82D as a rotor to a maximum degree with the inner coil bodies 94A to 94C and the outer coil bodies 95A to 95C at hollow portions of the inner coil bodies 94A to 94C and the outer coil bodies 95A to 95C, by which a structure with small inertia is formed as a rotor, thus increasing response speed.

Furthermore, by forming the respective inner coil bodies 94A to 94C and the respective outer coil bodies 95A to 95C into a roughly trapezoid or bowed shape, a space for arranging the commutators 93A to 93F and the brushes 78A , 78B can be ensured, thus achieving prevention of motor enlargement.

Figures 8A to 8C illustrates a tructural view of a DC motor according to a fourth embodiment of the present invention. Figure 8 illustrates a brush type coreless motor having rotating coil, Figure 8A is a structural view of the DC motor from the bottom without a brush base, Figure 8B is a cross-sectional view of Figure 8A through D-D and Figure 8C is a structural view of the brush base.

As illustrated in Figures 8A to 8C, the DC motor 101 is disposed, at angle of 90 degrees, adjacent to commutators 93A to 93F and the respective inner copil bodies 94A to 94C and the respective outer coil bodies 95A to 95C are connected to the commutators 93A to 93F so as to meet each other. Moreover, the commutators 93A and 93D, the commutators 93B and 93E and the commutators 93C and 93F are connected respectively. The other configurations are the same as for the DC motor 71 illustrated in Figures 6A to 6C, descriptions of which are omitted.

Figures 9A and 9B illustrate descriptive views of driving timings of a DC motor illustrated in Figures 8A to 8C, Figure 9A is a descriptive view illustrating commutators and brushes and Figure 9B is a timing chart of driving signals to respective coil bodies. In Figure 9A, the two brushes 78A, 78B are made adjacent to the commutators 93A to 93F at an angle of 90 degrees so as to abut thereagainst.

In the DC motor 101, as illustrated in Figure 9B, for example, positive and negative voltages are applied to the brushes 78A, 78B, so that driving power is alternately supplied to between a-phase and b-pahse (I, II) of the commutators 93A, 93D (inner coil body 94A, outer coil 95B) and the commutators 93B, 93E (inner coil body 94B, outer coil 95C), between a phase and c-pahse (I, III) of the commutators 93A, 93D (inner coil body 94A, outer coil 95B) and the commutators 93C, 93F (inner coil body 94C, outer coil 95A) and between b-phase and c-pahse (II, III) of the commutators 93B, 93E (inner coil body 94B, outer coil 95C) and the commutators 93C, 93F (inner coil body 94C, outer coil 95A), respectively, thus rotating the rotor 73.

That is, the respective inner coil bodies 94A to 94C and the respective outer coil bodies 95A to 95C, as illustrated in Figure 4A, are star-connected, of which phases facing each other are connected to each other to form three pairs of coil bodies. These phases are respectively connected to the commutators 93A to 93F of six segments, that is, to the commutators 93A, 93D, the commutators 93B, 93E and commutators 93C, 93F respectively,the respective inner coil bodies 94A to 94C and the respective outer coil bodies 95A to 95C switch electric currents four times respectively each time the rotor 73 makes one turn, thus achieving weight reduction and energy saving with high coil control efficiency.

Even in the case of an outer rotor type coreless motor with brush having commutators of six segments, the armature coil is constituted of the inner coil bodies 94A to 94C and the outer coil bodies 95A to 95C, of which phases are connected to each other, so that a minimum space can increase the number of windings of the armature coil as well as the number of generated magnetic fluxes. Thus, size and thickness reductions can be attained. Especially, even if the armature coil has a double structure as described above, by making the peripheral side surface of the inner coil group externally flush with the peripheral side surface of the outer coil group, a gap between the housing and the magnet can be made the same as for a conventional single structure using only the inner coil group, thus improving motor efficiency and generated torque.

The magnets 82A to 82D as a rotor at hollow portions of the inner coil bodies 94A to 94C and the outer coil bodies 95A to 95C have such a shape as to be enveloped with the inner coil bodies 94A to 94C and the outer coil bodies 95A to 95C to the maximum degree, respectively, and is formed into a structure having an inertia small enough to work as rotor, thus improving response speed.

Moreover, by forming the respective coil bodies 94A to 94C and the respective outer coil bodies 95A to 95C into a roughly trapezoidal or bowed shape, a space for arranging the commutators 93A to 93F and the brushes 78A, 78B can be ensured, thereby preventing motor enlargement.

The respective embodiments described above are described with the magnets serving as four poles, however, can be applied to DC motors equipped with the magnets of a larger number of poles 2n (n, two or larger integral number). In the respective embodiments above, a case where the inner coil group and the outer coil group are constituted of three inner coil bodies and three outer coil bodies respectively is described, however, such a structure that two or more inner coil bodies and two or more outer coil bodies are shifted from each other by a prescribed angle in a double arrangement (especially, the peripheral side surfaces of the inner coil group and the outer coil group are set at the identical outer periphery.) is also applicable. This can make the number of segments of commutators provided in the DC motor with brush the total number of the inner coil bodies and the outer coil bodies.

### Industrial Applicability

The DC motor of the present invention, basically constituted of magnets and armature coils, is applicable to a variety of parts ranging from very small-sized parts used in a clock or the like to medium-sized and large-sized parts used in a power system for a power assisted bicycle, an electric vehicle or the like.

## Claims

1. A DC motor having magnets as a main source for generating a magnetic flux and armature coils as a main source for generating a torque and using either thereof as a rotor,
wherein the armature coil comprises
an inner coil group formed by arranging, parallel with each other, a prescribed number of hollow inner coil bodies of a prescribed shape wound with a conductor of a prescribed number of turns on peripheral side surfaces of a virtual disc or a disc-shaped core; and
an outer coil group formed by arranging, parallel with each other, a prescribed number of hollow outer coil bodies of a prescribed shape wound with a conductor of a prescribed number of turns on peripheral side surfaces with the inner coil group taken as a virtual disc while covering the inner coil group.

2. The DC motor according to claim 1, wherein the peripheral side surface of the inner coil group is made externally flush with the peripheral side surface of the outer coil group.

3. The DC motor according to claim 1 or 2, wherein the respective inner coil bodies and the respective coil bodies are formed into a hollow and roughly trapezoidal or a hollow and arrowed shape, each of the corresponding inner coil bodies is arranged at intervals of 120 degrees, and wherein each of the corresponding outer coil bodies is shifted from each of the corresponding coil bodies by 60 degrees and arranged at intervals of 120 degrees.

4. The DC motor according to any one of claims 1 to 3, wherein phases facing between the inner coil bodies and the outer coil bodies are connected in series or in parallel to each other, each of which is star-connected.

5. The DC motor according to any one of claims 1 to 4, wherein, in a case where the armature coil portion is made to serve as a rotor, the DC motor includes commutators adaptable to the respective inner coil bodies and the respective outer coil bodies and four brushes arranged at intervals of 90 degrees for the respective commutators.

6. The DC motor according to any one of claims 1 to 4, wherein, in a case where the armature coil portion is made to serve as a rotor, the DC motor includes commutators adaptable to respective coil bodies formed by star connecting the respective inner coil bodies and the respective outer coil bodies and two brushes arranged at intervals of 90 degrees for the respective commutators.
